# EUROPÄISCHE PATENTSCHRIFT

(11) **EP 2 130 042 B1**
(45) Veröffentlichungstag und Bekanntmachung des Hinweises auf die Patenterteilung: **17.10.2012**
(21) Anmeldenummer: 08716131.1
(22) Anmeldetag: 29.02.2008
(51) Int. Cl.: G01N 33/487, B65D 47/08, B65D 83/08

(54) **Behälter für mehrere Teststreifen**
Container for a plurality of test strips
Contenant pour bandelettes réactives multiples

(30) Priorität: 29.03.2007 DE 102007015100
(43) Veröffentlichungstag der Anmeldung: 09.12.2009
(73) Patentinhaber: Merck Patent GmbH, 64293 Darmstadt (DE)
(72) Erfinder: MUELLER, Peter, 64347 Griesheim (DE); LANGKAU, Horst, 64291 Darmstadt (DE); WAGNER, Berthold, 60489 Frankfurt am Main (DE); WIELAND, Gerhard, 64625 Bensheim (DE); STORK, Norbert, 64291 Darmstadt (DE); BERNHARD, Stefan, 64711 Erbach (DE)
(86) Internationale Anmeldenummer: PCT/EP2008/001607
(87) Internationale Veröffentlichungsnummer: WO 2008/119421

(56) Entgegenhaltungen:
- EP-A- 0 167 783
- DE-A1- 19 909 426
- DE-U1- 8 800 462

## Beschreibung

Die Erfindung betrifft einen Behälter für mehrere Teststreifen, insbesondere für streifenförmige Indikatorstäbchen mit einem Gehäuseunterteil und mit einem formschlüssig mit dem Gehäuseunterteil in Eingriff bringbaren Gehäuseoberteil, wobei das Gehäuseoberteil eine Öffnung mit einer schwenkbar gelagerten Verschlussklappe aufweist und wobei die Verschlussklappe (14) längs ihres Umfangs mittels mindestens einem abreißbaren Verbindungssteg (25) mit dem Gehäuseoberteil (3) verbunden ist.

Behälter für streifenförmige Indikatorstäbchen sind in der Praxis seit langem bekannt. Diese Behälter bestehen üblicherweise aus einem quaderförmigen, einseitig offenen Gehäuseunterteil und einem ebenfalls quaderförmigem, einseitig offenen Gehäuseoberteil, wobei das Gehäuseoberteil auf das Gehäuseunterteil aufsteckbar ist, so dass ein allseitig geschlossener Behälter gebildet wird. Das Gehäuseoberteil wird kraftschlüssig auf dem Gehäuseunterteil gehalten.

Die Abmessungen des Gehäuseunterteils und des Gehäuseoberteils sind an die Abmessungen der streifenförmigen Indikatorstäbchen angepasst. Der Deckel sowie die einander gegenüberliegenden Seitenflächen des Gehäuseoberteils und der Boden sowie die gegenüberliegenden Seitenflächen des Gehäuseunterteils sind an die Breite der streifenförmigen Indikatorstäbchen angepasst. Der Behälter weist üblicherweise ein der Länge nach mehr als die Hälfte der darin gelagerten Indikatorstäbchen bedeckendes Gehäuseunterteil und ein deutlich kleineres Gehäuseoberteil auf.

Derartige Behälter sind schnell und kostengünstig herstellbar. Sie können in einfacher Weise automatisiert befüllt und verschlossen werden.

Derartige Behälter weisen jedoch keinen Originalitätsschutz auf. Es kann nicht festgestellt werden, ob der Behälter schon einmal geöffnet war und wieder verschlossen wurde. Sofern der Inhalt des Behälters einen Originalitätsschutz erforderlich macht, muss entweder der Behälter beispielsweise vollständig in eine aufreißbare Folienumhüllung eingebracht werden oder aber jeder einzelne Teststreifen mit einem Originalitätsschutz, beispielsweise mit einer aufreißbaren Umhüllung versehen werden, die bei dem erstmaligen Benutzen entfernt werden muss und nicht wieder angebracht werden kann. Ein derartiger Originalitätsschutz ist materialaufwändig und erfordert die gesonderte Anbringung einer Umhüllung, was mit zusätzlichen Kosten verbunden ist.

Auch die Handhabung derartiger Behälter wird oftmals als unpraktisch empfunden, da zur Entnahme eines Indikatorstäbchens das Gehäuseoberteil vollständig abgezogen werden muss und dann alle Indikatorstäbchen aus dem einseitig offenen Gehäuseunterteil herausfallen können.

Es sind verschiedene Ausführungen von Behältern bekannt (beispielsweise US 2005/0281706 A1, EP 0 270 868 B1, EP 0 640 393 B1, WO 2006/009534 A1, US 2006/0118570 A1), die in einfacher Weise die Entnahme eines einzelnen Teststreifens ermöglichen sollen. Der hierfür erforderliche konstruktive Aufwand ist jedoch sehr hoch. Derartige Entnahmevorrichtungen können zumindest teilweise leicht beschädigt werden und weisen nicht selten Fehlfunktionen auf, so dass kein Teststreifen mehr entnommen werden kann oder der Teststreifen beschädigt aus dem Behälter ausgebracht wird. Die bekannten Behälter, die als Teststreifenspender benutzt werden können, weisen zudem oftmals keinen Originalitätsschutz auf.

Es sind andere Behälter bekannt (beispielsweise EP 1 167 221 A1, US 4,934,556, DE 32 44 459 C2, EP 0 618 146 A2, DE 196 53 065 A1), die regelmäßig eine Verschlusskappe aufweisen, die über ein separates Abreißelement mit dem Behälter verbunden ist. Erst nach dem Heraustrennen des Abreißelements kann die Verschlusskappe geöffnet und wieder verschlossen werden. Das Abreißelement muss dabei regelmäßig vollständig von der Verschlusskappe und dem Behälter gelöst und entsorgt werden, was insbesondere bei einer Verwendung in chemischpharmazeutischen Laborräumen oder an Arbeitsplätzen, die erhöhte Anforderungen an Reinheit und Entsorgung stellen, mit einem nicht unerheblichen Aufwand verbunden sein kann. Auch bei derartigen Behältern ist der Herstellungsaufwand hoch. Die Behälter eignen sich nicht oder nur bedingt zur Aufnahme von Teststreifen. Eine einfache und zuverlässige Entnahme der Teststreifen ist nur selten möglich.

Bei einem Behälter (US 4,724,977) ist das Gehäuseoberteil formschlüssig mit dem Gehäuseunterteil verbindbar und weist im Bereich des Deckels des Gehäuseoberteils einen flachen, wieder verschließbaren Verschluss sowie einen unmittelbar darunter angeordneten, heraustrennbaren Verschluss auf. Bei einem erstmaligen Öffnen des Behälters muss zunächst der schwenkbar angeordnete, wieder verschließbare Verschluss geöffnet und dann der darunter befindliche heraustrennbare Verschluss herausgetrennt und entnommen werden. Der heraustrennbare Verschluss lässt sich nicht mehr in das Gehäuseoberteil einsetzen. Der Behälter kann nach seiner Benutzung durch den wieder verschließbaren Verschluss erneut verschlossen werden.

Um ein unerwünschtes Öffnen des Behälters durch das voneinander Lösen des Gehäuseoberteils von dem Gehäuseunterteil zu verhindern, ist vorgesehen, dass das Gehäuseoberteil eine wulstartige Verdickung des Gehäuseunterteils umgreift und aus einem ausreichend formstabilen Material hergestellt ist, so dass das Gehäuseoberteil nicht von dem Gehäuseunterteil abgezogen und erneut damit verbunden werden kann, ohne dass dies zu einer Beschädigung des Behälters führt. Der Aufwand für die Herstellung des komplex geformten Gehäuseoberteils sowie für die Befestigung des Gehäuseoberteils auf dem Gehäuseunterteil ist erheblich. Eine einfache Handhabung dieses Behälters bei der Entnahme von Teststreifen wird durch die nach innen vorspringenden Ausformungen entlang des Umfangs des Verschlusses in dem Gehäuseoberteil erschwert, die nach dem Heraustrennen des heraustrennbaren Verschlusses in dem Gehäuseoberteil verbleiben und hinter denen sich einzelne Teststreifen verhaken können.

Bei einem Behälter der Eingangs genannten Gattung (EP 0 167 783 A1) ist die Verschlusskappe längs ihres Umfangs mittels abreissbarer Verbindungsstege mit dem Gehäuseoberteil verbunden. Das Gehäuseoberteil weist mehrere vorspringende Rastelemente auf, die mit daran angepassten Ausnehmungen des Gehäuseoberteils in Eingriff bringbar sind. Die Entnahme einzelner Teststreifen wird allerdings dadurch erschwert, dass die Entnahmeöffnung in der Verschlusskappe durch einen wulstartigen Vorsprung gegenüber dem angrenzenden Bereich des Gehäuseoberteils abgegrenzt ist. Die Entnahmeöffnung befindet sich in einem Deckelbereich des Gehäuseoberteils beabstandet zu den Seitenwänden, so dass ein Teststreifen nicht ohne weiteres durch die Entnahmeöffnung entnommen werden kann.

Aufgabe der vorliegenden Erfindung ist es demzufolge, einen Behälter für mehrere Teststreifen so auszugestalten, dass der Behälter mit einfachen Mitteln mit einem Originalitätsschutz versehen ist und gleichzeitig eine einfache Handhabung bzw. Entnahme eines Teststreifens ermöglicht.

Diese Aufgabe wird erfindungsgemäß dadurch gelöst, dass an dem Gehäuseunterteil mehrere vorspringende Rastelemente angeordnet sind, die mit daran angepassten Ausnehmungen des Gehäuseoberteils in Eingriff bringbar sind, dass die mit der Verschlussklappe verschließbare Öffnung sowohl einen Bereich eines Gehäusedeckels als auch einen Bereich einer Seitenwand des Gehäuseoberteils umfasst und dass die Verschlussklappe schwenkbar an dem Gehäusedeckel des Gehäuseoberteils gelagert ist und eine Ecke des Gehäuseoberteils bildet, wobei die Länge der Verschlussklappe längs des Gehäusedeckels geringer als die Länge der Verschlussklappe längs der Seitenwand des Gehäuseoberteils ist.

Die vorspringenden Rastelemente an dem Gehäuseunterteil werden bei einem Aufschieben des Gehäuseoberteils auf das Gehäuseunterteil mit den daran angepassten Ausnehmungen in dem Gehäuseoberteil in Eingriff gebracht und bilden eine nicht mehr zerstörungsfrei lösbare Rastverbindung. Die Verschlussklappe ist über mindestens einen abreißbaren Verbindungssteg so mit dem Gehäuseoberteil verbunden, dass bei einem erstmaligen Öffnen der Verschlussklappe der mindestens eine Verbindungssteg abgerissen wird und dadurch das bereits erfolgte Öffnen der Verschlussklappe zuverlässig anzeigt. Indem das Gehäuseoberteil nicht zerstörungsfrei lösbar mit dem Gehäuseunterteil verbunden ist und die abreißbaren Verbindungsstege ein erstmaliges Öffnen der in dem Gehäuseoberteil angeordneten Verschlussklappe anzeigen, wird mit konstruktiv einfachen Mitteln ein zuverlässiger Originalitätsschutz ermöglicht.

Die vorspringenden Rastelemente und die daran angepassten Ausnehmungen lassen sich ebenso wie der mindestens eine abreißbare Verbindungssteg einfach und kostengünstig herstellen, ohne dass zusätzliches Material oder ein erhöhter Aufwand während der Herstellung erforderlich werden. Eine automatisierte Befüllung wird nicht erschwert, da das Gehäuseoberteil bei einer geeigneten Ausbildung der vorspringenden Rastelemente ohne merklich größeren Kraftaufwand mit dem Gehäuseunterteil verbunden werden kann. Die äußeren Abmessungen des Gehäuseoberteils sowie des Gehäuseunterteils müssen sich nicht notwendigerweise merklich von den Abmessungen der bereits aus der Praxis bekannten Behälter für streifenförmige Indikatorstäbchen unterscheiden, so dass kein größerer Aufwand bei einer Umstellung von den bereits aus der Praxis bekannten Behältern ohne Originalitätsschutz auf die vorangehend beschriebenen Behälter mit Originalitätsschutz anfällt.

Die mit der Verschlussklappe verschließbare Öffnung umfasst sowohl einen Bereich eines Gehäusedeckels als auch einen Bereich einer Seitenwand des Gehäuseoberteils. Es hat sich gezeigt, dass die Entnahme eines einzelnen Teststreifens erheblich erleichtert wird, wenn die Öffnung nicht ausschließlich im Bereich des Gehäusedeckels und damit an der Oberseite des Behälters angeordnet ist. Indem die geöffnete Verschlussklappe auch einen Teil der Seitenwand des Gehäuseoberteils frei gibt kann für eine Entnahme eines Teststreifens der Behälter leicht gekippt so gehalten werden, dass ein Endbereich eines Teststreifens sowohl von oben als auch von der Seite frei zugänglich ist und der Teststreifen leicht ergriffen und entnommen werden kann. Ein vollständiges Umdrehen bzw. auf den Kopf stellen des Behälters, wie es allein im Bereich des Gehäusedeckels angeordnete Öffnungen notwendig machen, ist nicht erforderlich. Die aus der Praxis bekannte Gefahr, dass bei der Entnahme eines einzigen Teststreifens mehrere oder gar alle Teststreifen aus dem Behälter herausfallen, kann dadurch vermindert oder vollständig unterbunden werden.

Um eine einfache Entnahme eines Teststreifens zu erleichtern, ist vorgesehen, dass die Verschlussklappe schwenkbar an dem Gehäusedeckel des Gehäuseoberteils gelagert ist und eine Ecke des Gehäuseoberteils bildet.

Erfindungsgemäß ist weiter vorgesehen, dass die Länge der Verschlussklappe längs des Gehäusedeckels geringer als die Länge der Verschlussklappe längs der Seitenwand des Gehäuseoberteils ist. Selbst bei einer rechtwinklig abstehenden geöffneten Verschlussklappe können unbeabsichtigt keine Teststreifen aus dem Behälter herausfallen, da der über die Seitenwand des Gehäuseunterteils bzw. des damit fluchtend angeordneten Gehäuseoberteils hinausragende Seitenwandbereich der Verschlussklappe eine Anschlagfläche bildet und die Teststreifen in dem Behälter zurückhält.

Vorzugsweise ist vorgesehen, dass die Rastelemente mehr als eine halbe Wandstärke des Gehäuseoberteils im Bereich um die Ausnehmungen, vorzugsweise mehr als eine dreiviertel Wandstärke und insbesondere vorzugsweise mehr als eine Wandstärke des Gehäuseoberteils vorspringen. In Abhängigkeit von dem verwendeten Material für das Gehäuseoberteil müssen die Rastelemente jedenfalls weit genug vorspringen, um das Gehäuseoberteil bei einem Ablösen von dem Gehäuseunterteil zu zerstören oder zumindest sichtbare Spuren zu hinterlassen, die ein gewaltsames Lösen dauerhaft anzeigen.

Dabei ist es zweckmäßig, wenn die Rastelemente auf der dem Gehäuseoberteil zugewandten Seite eine schräg verlaufende, das Aufstecken des Gehäuseoberteils erleichternde Gleitfläche und auf der dem Gehäuseunterteil zugewandten Seite eine senkrecht zur Oberfläche vorspringende Anschlagsfläche aufweisen.

In vorteilhafter Weise sind die Rastelemente im Bereich von Seitenkanten des Gehäuseunterteils angeordnet. Auf diese Weise wird verhindert, dass beispielsweise unter Zuhilfenahme eines flachen Hilfsmittels eine Seitenfläche des Gehäuseoberteils mit den darin angeordneten Ausnehmungen von dem Gehäuseunterteil abgehoben wird, um den Eingriff der Rastelemente mit den Ausnehmungen zu lösen und das Gehäuseoberteil zerstörungsfrei von dem Gehäuseunterteil zu trennen.

Um ein zerstörungsfreies Ablösen des Gehäuseoberteils von dem Gehäuseunterteil weiter zu erschweren ist vorgesehen, dass die Wandstärke des Gehäuseunterteils im Bereich der Überdeckung durch das Gehäuseoberteil um mindestens die Wandstärke des Gehäuseoberteils reduziert ist. Ist die Einengung des Gehäuseunterteils an die Wandstärke des Gehäuseoberteils angepasst, so kann das Gehäuseoberteil auf das Gehäuseunterteil derart aufgeschoben, bzw. aufgesteckt werden, dass die Außenflächen des Gehäuseoberteils fluchtend und fugenlos anschließend zu den jeweils zugeordneten Außenflächen des Gehäuseunterteils ausgerichtet und angeordnet sind. Dadurch werden Spalten oder Fugen und vorspringende Ausformungen vermieden, die ein mechanisch erzwungenes Auftrennen und voneinander Lösen des Gehäuseoberteils von dem Gehäuseunterteil erleichtern oder ermöglichen könnten.

Es ist ebenfalls denkbar, dass sowohl die Wandstärke des Gehäuseunterteils als auch die Wandstärke des Gehäuseoberteils im Wesentlichen vergleichbar sind und im Bereich der Überdeckung jeweils aneinander angepasst vermindert sind. Das Gehäuseoberteil kann dann auf das Gehäuseunterteil aufgeschoben, bzw. aufgesteckt werden, so dass sowohl die Außenflächen als auch die Innenflächen des Gehäuseoberteils und des Gehäuseunterteils im Wesentlichen fluchtend und fugenlos aneinander stoßen. Die Wandstärke des Behälters ist dann durchgehend gleichbleibend ausgestaltet. Unerwünschte Stufen und vorspringende Kanten werden sowohl an den Innenflächen als auch an den Außenflächen vermieden.

Um eine einfache und kostengünstige Herstellung zu ermöglichen und gleichzeitig eine zuverlässige Originalitätssicherung der Verschlussklappe zu gewährleisten ist in vorteilhafter Weise vorgesehen, dass die Verschlussklappe an zwei gegenüberliegenden Seiten jeweils mindestens einen abreißbaren Verbindungssteg aufweist. Die Verschlussklappe weist zweckmäßigerweise einen im Wesentlichen rechteckigen Umfang bzw. Rand auf, der auch die Öffnung in dem Gehäuseoberteil begrenzt. Die Verschlussklappe ist beispielsweise mittels eines Filmscharniers längs einer Seite der Verschlussklappe schwenkbar an dem Gehäuseoberteil gelagert. An den zu beiden Seiten an das Filmscharnier angrenzenden gegenüberliegenden Seiten ist jeweils ein abreißbarer Verbindungssteg ausgebildet, der die Verschlussklappe mit dem Gehäuseoberteil verbindet und der erst nach dessen Abreißen die Verschlussklappe frei gibt und öffnen lässt.

An der dem Filmscharnier gegenüberliegenden Seite der Verschlussklappe kann eine klemmende oder formschlüssige Verschlusseinrichtung vorgesehen sein, welche die Verschlussklappe lösbar in einer geschlossenen Stellung an dem Gehäuseoberteil festlegt bzw. zurückhält. In einfacher Weise kann dies dadurch bewirkt werden, dass eine an der Verschlussklappe ausgeformte Rastzunge bei geschlossener Verschlussklappe den elastisch verformbaren Rand der Öffnung des Gehäuseoberteils hintergreift und die Verschlussklappe in der geschlossenen Position zurückhält. Es ist auch denkbar, die an Stelle des Randes der Öffnung des Gehäuseoberteils oder zusätzlich hierzu die Rastzunge der Verschlussklappe elastisch verformbar auszugestalten oder eine Rastzunge an dem Rand der Öffnung des Gehäuseoberteils auszubilden.

Die Form der abreißbaren Verbindungsstege kann nahezu beliebig vorgegeben und an die jeweiligen Anforderungen während der Herstellung des Gehäuseoberteils, des verwendeten Materials sowie der gewünschten Originalitätssicherung angepasst werden. Es hat sich als besonders vorteilhaft herausgestellt, wenn die abreißbaren Verbindungsstege als schmale und relativ zur Wandstärke verjüngte Übergänge einer ansonsten um den Rand der Verschlussklappe bildenden Trennungsfuge ausgebildet sind. Es können natürlich auch mehrere abreißbare Verbindungsstege vorgesehen sein, die entweder gleichmäßig oder unregelmäßig beabstandet die Verschlussklappe mit dem Gehäuseunterteil verbinden.

Ein Ausführungsbeispiel des Erfindungsgedankens wird nachfolgend anhand der Zeichnung näher erläutert. Es zeigt:
Fig. 1 eine Seitenansicht eines Behälters für mehrere Teststreifen,
Fig. 2 eine andere Seitenansicht des in Fig. 1 dargestellten Behälters,

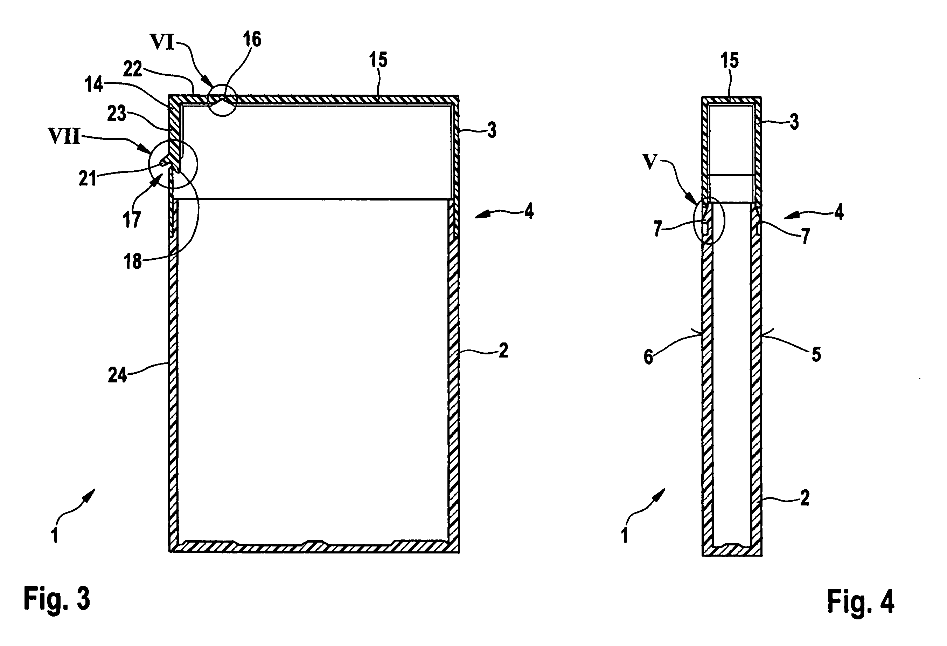

Fig. 3 eine Schnittansicht des in den Fig. 1 und 2 dargestellten Behälters längs der Linie III-III in Fig. 2,
Fig. 4 eine Schnittansicht des in den Fig. 1 und 2 dargestellten Behälters längs der Linie IV-IV in Fig. 1,
Fig. 5 einen vergrößert dargestellten Teilbereich gemäß V in Fig. 4,
Fig. 6 einen vergrößert dargestellten Teilbereich gemäß VI in Fig. 3
Fig. 7 einen vergrößert dargestellten Teilbereich gemäß VII in Fig. 3,
Fig. 8 einen vergrößert dargestellten Teilbereich gemäß VIII in Fig. 1,
Fig. 9 eine Schnittansicht des in Fig. 8 dargestellten Teilbereichs längs der Linie IX-IX und
Fig. 10 eine der Ansicht gemäß Fig. 9 entsprechende Ansicht einer alternative Ausgestaltung eines Gehäuseoberteils des in Fig. 1 dargestellten Behälters und
Fig. 11 eine Seitenansicht des in Fig. 1 dargestellten Behälters mit einer geöffneten Verschlussklappe.

Der in den Figuren dargestellte Behälter 1 für mehrere Teststreifen, insbesondere für eine größere Anzahl von streifenförmigen Indikatorstäbchen, weist ein im Wesentlichen quaderförmiges, einseitig offenes Gehäuseunterteil 2 und ein ebenfalls im Wesentlichen quaderförmiges, einseitig offenes Gehäuseoberteil 3 auf. Das Gehäuseoberteil 3 ist auf das Gehäuseunterteil 2 aufgesteckt und rastend mit diesem verbunden, so dass ein allseitig geschlossener Behälter 1 für darin befindliche, in den Figuren nicht dargestellte Teststreifen gebildet wird.

Das Gehäuseunterteil 2 weist in einem dem Gehäuseoberteil zugewandten Randbereich 4 eine verminderte Wandstärke auf, so dass das Gehäuseoberteil 3 mit einer daran angepassten Wandstärke so auf das Gehäuseunterteil 2 aufgeschoben werden kann, dass die Außenflächen des Gehäuseunterteils 2 und des Gehäuseoberteils 3 fluchtend und bündig aneinander stoßen.

Das Gehäuseunterteil 2 weist auf einer Vorderseite 5 und einer Rückseite 6 jeweils in dem Randbereich 4 angeordnete, nach außen vorspringende Rastelemente 7 auf. Die Rastelemente 7 weisen auf einer dem Gehäuseoberteil 3 zugewandten Seite eine schräg verlaufende Gleitfläche 8 und auf der dem Gehäuseunterteil 2 zugewandten Seite einer senkrecht zur Vorderseite 5 bzw. zur Rückseite 6 vorspringende Anschlagsfläche 9 auf. Diese Formgebung der Rastelemente 7 bewirkt, dass das Gehäuseoberteil 3 mit verhältnismäßig geringem Kraftaufwand auf das Gehäuseunterteil 2 aufgeschoben werden kann, bis die Rastelemente 7 mit daran angepassten Ausnehmungen 10 in dem Gehäuseoberteil 3 in Eingriff treten.

Sobald das Gehäuseoberteil 3 vollständig auf das Gehäuseunterteil 2 aufgeschoben ist, bilden die senkrecht zur Oberfläche ausgerichteten Anschlagsflächen 9 der Rastelemente 7 in Verbindung mit den Randbereichen der Ausnehmungen 10 eine formschlüssige Verbindung, die auch bei erheblicher Krafteinwirkung nicht mehr zerstörungsfrei gelöst werden kann.

Da die Rastelemente 7 bzw. die daran angepassten Ausnehmungen 10 jeweils im Bereich von Seitenkanten 11, 12 des Gehäuseunterteils 2 bzw. des Gehäuseoberteils 3 angeordnet sind, können die Vorderseite 5 oder die Rückseite 6 des Gehäuseoberteils nicht derart verformt und von der Vorderseite 5 bzw. der Rückseite 6 des Gehäuseunterteils abgehoben werden, so dass die Randbereiche der Ausnehmungen 10 vollständig von den Anschlagsflächen 9 der Rastelemente 7 getrennt würden und das Gehäuseoberteil 3 ungehindert von dem Gehäuseunterteil 2 abgezogen werden könnte.

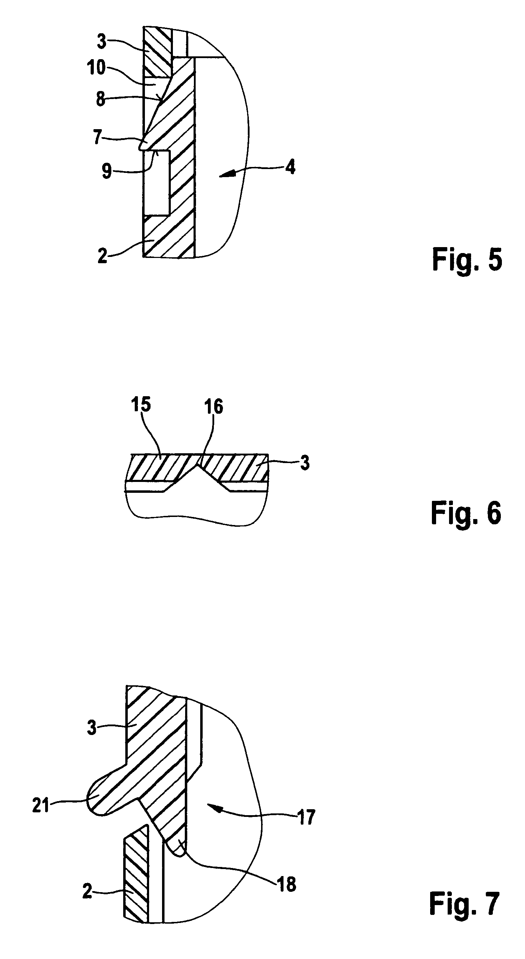

Wie insbesondere aus Fig. 5 ersichtlich ist, ragen die Rastelemente 7 im Bereich der Anschlagsfläche 9 mehr als eine Wandstärke des Gehäuseoberteils im Bereich um die Ausnehmungen 10 vor. Auf diese Weise kann ein unerwünschtes Ablösen des Gehäuseoberteils 3 von dem Gehäuseunterteil 2 zuverlässig vermieden werden. In Abhängigkeit von dem jeweils verwendeten Material insbesondere für das Gehäuseoberteil 3 könnte es auch ausreichen, wenn die Rastelemente 7 lediglich weniger als eine Wandstärke vorspringen.

Das Gehäuseoberteil 3 weist eine Ecke 13 des Gehäuseoberteils 3 bildende Verschlussklappe 14 auf. Die Verschlussklappe 14 ist im Bereich eines Gehäusedeckels 15 an einer Oberseite des Gehäuseoberteils 3 schwenkbar gelagert. Zu diesem Zweck ist die Verschlussklappe 14 mit dem Gehäusedeckel 15 einstückig ausgeführt und über eine in Fig. 6 vergrößert dargestellte Wandstärkenverminderung 16 verbunden, die ein Filmscharnier bildet. Durch die Wandstärkenverminderung 16 wird ein schmaler Bereich des Gehäusedeckels 15 mit verminderter Steifigkeit, bzw. erhöhter Elastizität erzeugt, der ein einfaches Verschwenken der Verschlussklappe 14 zum Öffnen und Verschließen des Behälters 1 ermöglicht.

An der der Wandstärkenverminderung 16 gegenüberliegenden Seitenkante 17 weist die Verschlusskappe 14 eine vorspringende Rastzunge 18 auf. Die Rastzunge 18 hintergreift in einem geschlossenen Zustand der Verschlussklappe 14 einen Rand 19 einer von der Verschlussklappe 14 bedeckten Öffnung 20 in dem Gehäuseoberteil 3. Mit der Rastzunge 18 wird die Verschlussklappe 14 in der geschlossenen Position zurückgehalten. Die Verschlussklappe 14 weist einen nach außen vorspringenden Griffbereich 21 auf, der ein Ergreifen und Öffnen der Verschlussklappe 14 erleichtert.

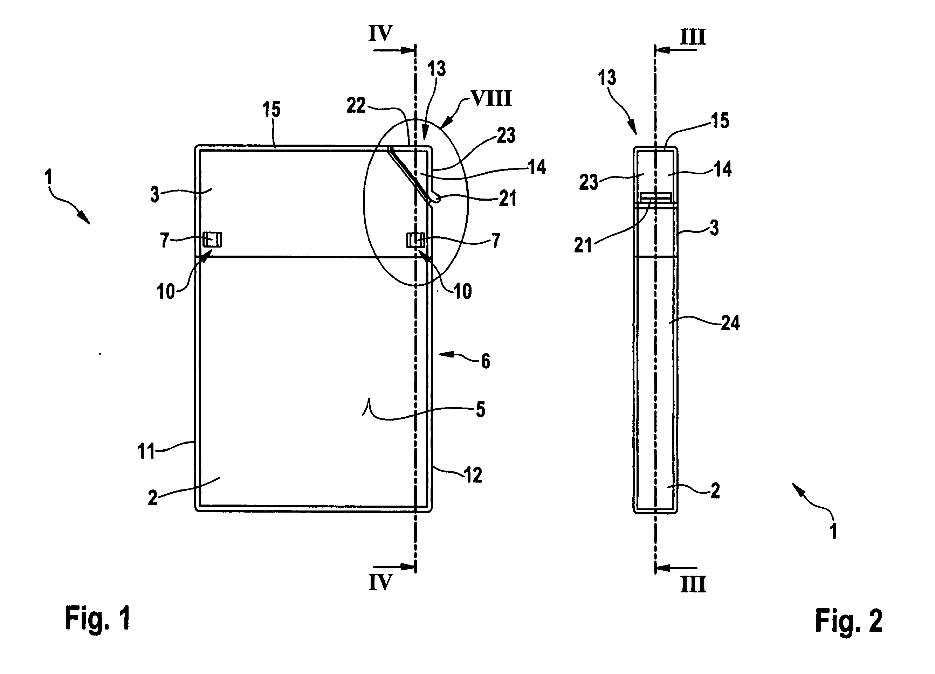

Wie aus Fig. 1 ersichtlich ist, bildet die Verschlussklappe 14 eine Ecke 13 des Gehäuseoberteils. Ein erster Abschnitt 22 der Verschlussklappe 14 längs des Gehäusedeckels 15 ist kürzer als ein zweiter Abschnitt 23 der Verschlussklappe 14, der sich längs einer schmalen Seitenwand 24 erstreckt. Wird die Verschlussklappe 14 rechtwinklig abstehend geöffnet, so erstreckt sich der längere zweite Abschnitt 23 mit dem Gehäusedeckel 15 fluchtend über die schmale Seitenwand 24 hinaus, so dass in dem Behälter 1 befindliche Teststreifen nicht herausfallen können, selbst wenn der Behälter 1 umgedreht und auf den Kopf gestellt wird.

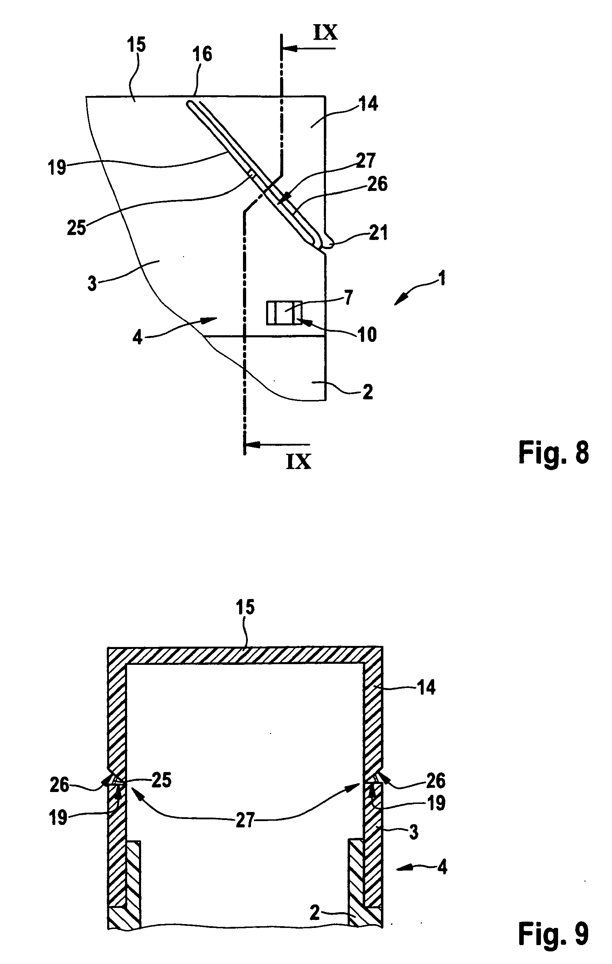

In den Fig. 8 und 9 ist zur Verdeutlichung der Behälter 1 im Bereich der Verschlussklappe 14 vergrößert und in seitlicher, bzw. geschnittener Ansicht dargestellt. Die Verschlussklappe 14 ist über zwei abreißbare Verbindungsstege 25 mit dem Rest des Gehäuseoberteils 3 verbunden. Um den Behälter 1 erstmalig zu öffnen, müssen die abreißbaren Verbindungsstege 25 abgerissen bzw. aufgetrennt werden, bevor die Verschlussklappe 14 von der geschlossenen Stellung in eine geöffnete Stellung verschwenkt werden kann. Die dann abgerissenen Verbindungsstege 25 lassen erkennen, dass der Behälter 1 bzw. die Verschlussklappe 14 bereits einmal geöffnet waren ein Originalitätsschutz für die in dem Behälter 1 befindlichen Teststreifen nicht mehr gewährleistet ist.

Ein unterer Rand 26 der Verschlussklappe 14 verjüngt sich spitz zulaufend. Zwischen dem unteren Rand 26 der Verschlussklappe 14 und dem Rand 19 der Öffnung 20 in dem Gehäuseoberteil 3 ist eine Trennfuge 27 ausgebildet. Die Verbindungsstege 25 sind einstückig in dem Gehäuseoberteil 3 ausgeformt und verbinden als schmaler Verbindungssteg 25 mit einer verringerten Wandstärke die Verschlussklappe 14 mit dem Rest des Gehäuseoberteils 3.

Die Wandstärke des Gehäuseunterteils 2 ist im Randbereich 4 der Überdeckung durch das Gehäuseoberteil 3 um exakt die Wandstärke des Gehäuseoberteils 3 reduziert. Das Gehäuseoberteil 3 kann derart auf das Gehäuseunterteil 2 aufgeschoben, bzw. aufgesteckt werden, dass die Außenflächen des Gehäuseoberteils 3 fluchtend und fugenlos anschließend zu den jeweils zugeordneten Außenflächen des Gehäuseunterteils 2 ausgerichtet und angeordnet sind. Dadurch werden Spalten oder Fugen sowie vorspringende Ausformungen vermieden, die ein mechanisch erzwungenes Auftrennen und voneinander Lösen des Gehäuseoberteils 3 von dem Gehäuseunterteil 2 erleichtern oder ermöglichen könnten.

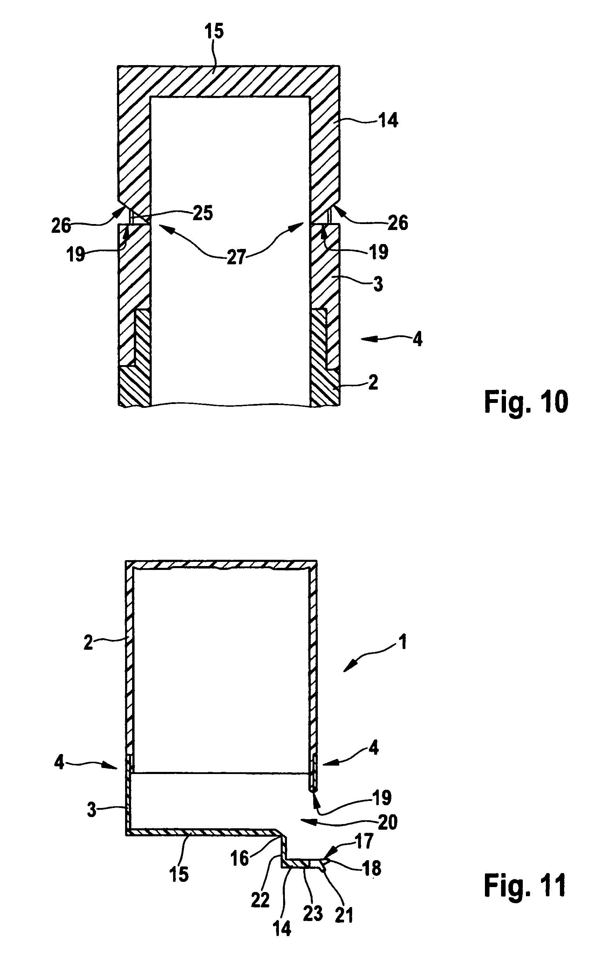

In Fig. 10 ist eine alternative Ausgestaltung des Gehäuseoberteils 3 dargestellt. Die Wandstärke des Gehäuseoberteils 3 entspricht der Wandstärke des Gehäuseunterteils 2. Im Randbereich 4 der Überdeckung sind sowohl die Wandstärke des Gehäuseunterteils 3 als auch die Wandstärke des Gehäuseoberteils 2 jeweils auf die Hälfte reduziert, so dass nicht nur die jeweiligen Außenflächen des Gehäuseoberteils 3 und des Gehäuseunterteils 2 fluchtend und fugenlos aneinanderstoßen, sondern auch die jeweiligen Innenflächen fluchtend und ohne vorspringende Kanten zueinander ausgerichtet und angeordnet sind.

In Fig. 11 ist zur Veranschaulichung der Behälter 1 mit geöffneter Verschlussklappe 14 auf dem Kopf stehend angeordnet dargestellt. Der zweite Abschnitt 23 der Verschlussklappe 14, der im rechtwinklig geöffneten Zustand der Verschlussklappe 14 parallel zum Gehäusedeckel 15 ausgerichtet ist, ragt über die schmale Seitenwand 24 hinaus und verhindert auch bei einem über Kopf angeordneten Behälter 1 und geöffneter Verschlussklappe 14, dass in dem Behälter 1 befindliche Teststreifen herausfallen. Da ein oder mehrere Teststreifen im Bereich der Verschlussklappe 14 teilweise herausrutschen und erst durch den zweiten Abschnitt 23 der Verschlussklappe 14 zurückgehalten werden liegen diese Teststreifen im Bereich der Öffnung 20 frei zugänglich auf der Verschlussklappe 14 auf und können einfach, schnell und zuverlässig einzeln entnommen werden.

Das Gehäuseoberteil 3 sowie das Gehäuseunterteil 2 sind jeweils vorzugsweise einstückig aus einem Kunststoff wie beispielsweise Polystyrol (PS) oder Polypropylen (PP) im Spritzgussverfahren hergestellt. Dabei ist es vorteilhaft, wenn das Gehäuseoberteil 3 aus einem elastischen Kunststoffmaterial wie beispielsweise Polypropylen (PP) oder Polyethylen (PE) hergestellt ist. Das Gehäuseunterteil 2 besteht zweckmäßigerweise aus einem Kunststoff mit einer ausreichenden Steifigkeit, bzw. Festigkeit, wie beispielsweise Polystyrol (PS). Um einem Benutzer einen Einblick auf den Inhalt des Behälters 1 zu erlauben können das Gehäuseunterteil 2 und/oder das Gehäuseoberteil 3 aus einem möglichst durchsichtigen Kunststoff hergestellt sein. Im Bereich der Rastelemente 7 kann das für das Spritzverfahren verwendete Werkzeug beweglich gelagerte Schieber aufweisen, um die bei dem Ausführungsbeispiel dargestellten Hinterschneidungen im Bereich der Rastelemente 7 herstellen zu können.

Die vorliegende Beschreibung ermöglicht es dem Fachmann, die Erfindung umfassend anzuwenden. Auch ohne weitere Ausführungen wird daher davon ausgegangen, dass ein Fachmann die obige Beschreibung im weitesten Umfang nutzen kann. Bei etwaigen Unklarheiten versteht es sich von selbst, die zitierten Veröffentlichungen und Patentliteratur heranzuziehen.

Zum besseren Verständnis und zur Verdeutlichung der Erfindung werden Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren; dieser Schutzbereich wird durch die Ansprüche definiert.

Sofern nicht ausdrücklich als erfindungswesentliches Merkmal gekennzeichnet versteht es sich für den Fachmann von selbst, dass die in den Ausführungsbeispielen dargestellten oder abgebildeten Abmessungen oder relativen Größenverhältnisse lediglich jeweils mögliche Ausgestaltungen des Erfindungsprinzips veranschaulichen sollen, ohne dass damit eine Einschränkung auf diese Abmessungen oder Größenverhältnisse verbunden wäre.

## Patentansprüche

1. Behälter (1) für mehrere Teststreifen, insbesondere für streifenförmige Indikatorstäbchen, mit einem Gehäuseunterteil (2) und mit einem formschlüssig mit dem Gehäuseunterteil (2) in Eingriff bringbaren Gehäuseoberteil (3), wobei das Gehäuseoberteil (3) eine Öffnung (2) mit einer schwenkbar gelagerten Verschlussklappe (14) aufweist und wobei die Verschlussklappe (14) längs ihres Umfangs mittels mindestens einem abreißbaren Verbindungssteg (25) mit dem Gehäuseoberteil (3) verbunden ist, **dadurch gekennzeichnet, dass** das Gehäuseunterteil (2) mehrere vorspringende Rastelemente (7) aufweist, die mit daran angepassten Ausnehmungen (10) des Gehäuseoberteils (3) in Eingriff bringbar sind, dass die mit der Verschlussklappe (14) verschließbare Öffnung (20) sowohl einen Bereich eines Gehäusedeckels (15) als auch einen Bereich einer Seitenwand (24) des Gehäuseoberteils (3) umfasst und dass die Verschlussklappe (14) schwenkbar an dem Gehäusedeckel (15) des Gehäuseoberteils (3) gelagert ist und eine Ecke (13) des Gehäuseoberteils (3) bildet, wobei die Länge der Verschlussklappe (14) längs des Gehäusedeckels (15) geringer als die Länge der Verschlussklappe (14) längs der Seitenwand (24) des Gehäuseoberteils (3) ist.

2. Behälter nach Anspruch 1, **dadurch gekennzeichnet, dass** die Rastelemente (7) mehr als eine halbe Wandstärke des Gehäuseoberteils (3) im Bereich um die Ausnehmungen (10), vorzugsweise mehr als eine dreiviertel Wandstärke und insbesondere vorzugsweise mehr als eine Wandstärke des Gehäuseoberteils (3) vorspringen.

3. Behälter nach Anspruch 1 oder Anspruch 2, **dadurch gekennzeichnet, dass** die Rastelemente (7) im Bereich von Seitenkanten (11, 2) des Gehäuseunterteils (2) angeordnet sind.

4. Behälter nach einem oder mehreren der vorangehenden Ansprüche 1 bis 3, **dadurch gekennzeichnet, dass** die Wandstärke des Gehäuseunterteils (2) im Bereich der Überdeckung durch das Gehäuseoberteil (3) um mindestens die Wandstärke des Gehäuseoberteils (3) reduziert ist.

5. Behälter nach einem oder mehreren der vorangehenden Ansprüche 1 bis 3, **dadurch gekennzeichnet, dass** sowohl die Wandstärke des Gehäuseunterteils (2) als auch die Wandstärke des Gehäuseoberteils (3) im Wesentlichen vergleichbar sind und im Bereich der Überdeckung jeweils aneinander angepasst vermindert sind.

6. Behälter nach einem oder mehreren der vorangehenden Ansprüche 1 bis 5, **dadurch gekennzeichnet, dass** die Verschlussklappe (14) an zwei gegenüberliegenden Seiten jeweils mindestens einen abreißbaren Verbindungssteg (25) aufweist.

7. Verwendung eines Behälters nach einem oder mehreren der Ansprüche 1 bis 6 zur Aufnahme und Lagerung von Teststreifen.

8. Verwendung eines Behälters nach Anspruch 7, **dadurch gekennzeichnet, dass** die Teststreifen streifenförmige Indikatorstäbchen sind.

## Claims

1. Container (1) for a plurality of test strips, in particular for strip-shaped indicator sticks, having a housing lower part (2) and having a housing upper part (3), which can be brought into mechanical engagement with the housing lower part (2), where the housing upper part (3) has an opening (2) with a sealing flap (14) mounted in a pivotable manner and where the sealing flap (14) is connected along its periphery to the housing upper part (3) by means of at least one tear-off connecting strip (25), **characterised in that** the housing lower part (2) has a plurality of projecting snap-in elements (7), which can be brought into engagement with matched recesses (10) of the housing upper part (3), **in that** the opening (20), which can be sealed by means of the sealing flap (14), encompasses both a region of a housing lid (15) and also a region of a side wall (24) of the housing upper part (3) and **in that** the sealing flap (14) is mounted in a pivotable manner on the housing lid (15) of the housing upper part (3) and forms a corner (13) of the housing upper part (3), where the length of the sealing flap (14) along the housing lid (15) is less than the length of the sealing flap (14) along the side wall (24) of the housing upper part (3).

2. Container according to Claim 1, **characterised in that** the snap-in elements (7) project by more than half of the wall thickness of the housing upper part (3) in the region around the recesses (10), preferably by more than three quarters of the wall thickness and particularly preferably by more than one wall thickness of the housing upper part (3).

3. Container according to Claim 1 or Claim 2, **characterised in that** the snap-in elements (7) are arranged in the region of side edges (11, 2) of the housing lower part (2).

4. Container according to one or more of the preceding Claims 1 to 3, **characterised in that** the wall thickness of the housing lower part (2) is reduced by at least the wall thickness of the housing upper part (3) in the region of the overlap by the housing upper part (3).

5. Container according to one or more of the preceding Claims 1 to 3, **characterised in that** both the wall thickness of the housing lower part (2) and also the wall thickness of the housing upper part (3) are essentially comparable and are each reduced matched to one another in the region of the overlap.

6. Container according to one or more of the preceding Claims 1 to 5, **characterised in that** the sealing flap (14) has at least one tear-off connecting strip (25) on each of two opposite sides.

7. Use of a container according to one or more of Claims 1 to 6 for the accommodation and storage of test strips.

8. Use of a container according to Claim 7, **characterised in that** the test strips are strip-shaped indicator sticks.

## Revendications

1. Conteneur (1) pour une pluralité de bandes de test, en particulier pour des sticks indicateurs en forme de bande, comportant une partie inférieure de logement (2) et comportant une partie supérieure de logement (3), laquelle peut être amenée en coopération mécanique avec la partie inférieure de logement (2), où la partie supérieure de logement (3) comporte une ouverture (2) munie d'un volet d'étanchéité (14) monté de façon pivotable et où le volet d'étanchéité (14) est connecté le long de sa périphérie à la partie supérieure de logement (3) au moyen d'au moins une bande de connexion enlevable par déchirement (25), **caractérisé en ce que** la partie inférieure de logement (2) comporte une pluralité d'éléments encliquetables en protubérance (7), lesquels peuvent être amenés en coopération avec des évidements conjugués (10) de la partie supérieure de logement (3), **en ce que** l'ouverture (20), qui peut être rendue étanche au moyen du volet d'étanchéité (14), englobe à la fois une région d'un couvercle de logement (15) et également une région d'une paroi latérale (24) de la partie supérieure de logement (3) et **en ce que** le volet d'étanchéité (14) est monté de façon pivotante sur le couvercle de logement (15) de la partie supérieure de logement (3) et forme un angle (13) de la partie supérieure de logement (3), où la longueur du volet d'étanchéité (14) le long du couvercle de logement (15) est inférieure à la longueur du volet d'étanchéité (14) le long de la paroi latérale (24) de la partie supérieure de logement (3).

2. Conteneur selon la revendication 1, **caractérisé en ce que** les éléments encliquetables (7) font saillie de plus de la moitié de l'épaisseur de paroi de la partie supérieure de logement (3) dans la région autour des évidements (10), de préférence de plus des trois quarts de l'épaisseur de paroi, de façon particulièrement préférable de plus d'une épaisseur de paroi de la partie supérieure de logement (3).

3. Conteneur selon la revendication 1 ou la revendication 2, **caractérisé en ce que** les éléments encliquetables (7) sont agencés dans la région de bords latéraux (11, 2) de la partie inférieure de logement (2).

4. Conteneur selon une ou plusieurs des revendications précédentes 1 à 3, **caractérisé en ce que** l'épaisseur de paroi de la partie inférieure de logement (2) est réduite d'au moins l'épaisseur de paroi de la partie supérieure de logement (3) dans la région du chevauchement par la partie supérieure de logement (3).

5. Conteneur selon une ou plusieurs des revendications précédentes 1 à 3, **caractérisé en ce que** l'épaisseur de paroi de la partie inférieure de logement (2) et également l'épaisseur de paroi de la partie supérieure de logement (3) sont toutes deux essentiellement comparables et sont chacune adaptées par réduction relative dans la région du chevauchement.

6. Conteneur selon une ou plusieurs des revendications précédentes 1 à 5, **caractérisé en ce que** le volet d'étanchéité (14) comporte au moins une bande de connexion enlevable par déchirement (25) sur chacun de deux côtés opposés.

7. Utilisation d'un conteneur selon une ou plusieurs des revendications 1 à 6 pour le logement et le stockage de bandes de test.

8. Utilisation d'un conteneur selon la revendication 7, **caractérisé en ce que** les bandes de test sont des sticks indicateurs en forme de bande.
